# FASCICULE DE BREVET EUROPEEN

(11) **EP 1 276 540 B1**
(45) Date de publication et mention de la délivrance du brevet: **03.03.2004**
(21) Numéro de dépôt: 01923778.3
(22) Date de dépôt: 10.04.2001
(51) Int. Cl.: A61N 7/02

(54) **SYSTEME DE MANIPULATION DE FLUIDE POUR APPAREIL DE THERAPIE**
FLUIDBEHANDLUNGSVORRICHTUNG FÜR EINEN THERAPEUTISCHEN APPARAT
FLUID MANIPULATING SYSTEM FOR THERAPY APPARATUS

(30) Priorité: 12.04.2000 FR 0004703
(43) Date de publication de la demande: 22.01.2003
(73) Titulaire: TECHNOMED MEDICAL SYSTEMS, 69120 Vaulx-en-Velin (FR)
(72) Inventeur: TARDY, Frédérique, Technomed Medical Systems, F-69120 Vaulx en Velin (FR); MARTIN, Yves, Technomed Medical Systems, F-69120 Vaulx en Velin (FR); BLANC, Emmanuel, Technomed Medical Systems, F-69120 Vaulx en Velin (FR)
(74) Mandataire: Cabinet Hirsch
(86) Numéro de dépôt international: PCT/FR2001/001090
(87) Numéro de publication internationale: WO 2001/078837

(56) Documents cités:
- DE-C- 19 509 005
- FR-A- 2 750 340
- US-A- 3 777 507
- US-A- 5 086 449

## Description

L'invention concerne les systèmes de manipulation de fluides, et plus spécifiquement de manipulation de fluides utilisés dans des appareils de thérapie. Elle concerne aussi un kit (ensemble de pièces préassemblées) pour la réalisation d'un tel système.

US-A-5 899 873 décrit un système de délivrance d'un fluide biologique, en l'espèce d'une solution de cardioplégie pour l'alimentation du coeur pendant une opération à coeur ouvert. Le système est constitué d'une pompe, qui reçoit d'une part du sang provenant d'une machine d'oxygénation, et d'autre part une solution cristalloïde provenant d'un réservoir. La pompe mélange ces deux fluides, et les transmet à un échangeur de chaleur; celui-ci reçoit par ailleurs une solution de potassium, ou des solutions d'additifs. Les fluides mélangés et portés à une température appropriée sont ensuite dirigés vers le coeur du patient. L'échangeur de chaleur de ce document est un ensemble monobloc, qui assure les fonctions de contrôle de température, de filtration, de séparation des gaz, et de mesure des paramètres du fluide de cardioplégie, comme la pression ou la température. Dans ce système, il n'y a pas de récupération du fluide; le débit de la pompe est ajusté en fonction de la pression mesurée par le capteur de pression.

US-A-5 984 893 décrit un système d'infusion rapide de fluide sanguin permettant de contrôler le taux d'hématocrite d'un patient. Le système comprend une source de fluide et une source de sang, sous la forme de sacs souples susceptibles d'être suspendus à des mats connus en soi. Deux pompes contrôlent le débit de fluide et de sang. Le mélange s'effectue dans une tubulure et est envoyé vers un échangeur de chaleur, un dégazeur, un détecteur de bulles et un capteur de température et de pression. Les pompes sont commandées par un contrôleur de remplacement du sang, qui contrôle le débit des pompes de sorte à maintenir un taux d'hématocrite constant dans le sang du patient. La section d'état de la technique de ce document décrit des dispositifs d'injection, comprenant un réservoir de cardiotomie, une pompe de circulation, un échangeur de chaleur et une trappe à bulles.

Ces deux documents montrent des systèmes en circuit ouvert, pour l'injection d'un fluide.

US-A-4 874 359 montre encore un système d'infusion rapide de fluide sanguin. Sont prévus de nouveau deux réservoirs de fluide et de sang, dont les sorties respectives sont mélangées dans un réservoir. Le mélange du réservoir est fourni à un détecteur de bulle, puis à une pompe péristaltique à capteur de débit. La sortie de la pompe est reliée à un échangeur de chaleur, puis à une trappe à bulles. La sortie d'air de la trappe à bulles est envoyée vers le réservoir, de sorte à pouvoir purger la trappe à bulles sans ouvrir l'infuseur sur l'extérieur. En sortie de la trappe à bulles vers le patient est prévu un connecteur en Y muni de capteurs de température et de pression; une sortie du Y part vers le patient, et l'autre sortie est une boucle de recirculation vers le réservoir.

Le système de ce document permet une circulation du fluide, mais uniquement avant qu'il ne soit injecté dans le patient.

US-A-4 186 565 décrit un système de perfusion pour la préservation d'organes comme les reins. Ce système comprend une cuve, et un circuit fermé de fluide; le circuit comprend, dans l'ordre, une pompe, une trappe à bulle, l'organe, un oxygénateur, un échangeur de chaleur, et de nouveau la pompe. L'objectif dans ce document est d'alimenter l'organe en fluide, pour assurer sa préservation dans la solution de la cuve.

Ces différents systèmes ne sont pas adaptés à la manipulation de fluide pour un appareil, dans lequel le fluide n'est pas destiné à être injecté, mais doit être récupéré ou circulé. Un exemple d'un tel appareil est l'appareil de traitement par hyperthermie de la prostate commercialisé par la demanderesse sous la marque Ablatherm, et qui est décrit dans les demandes de brevet FR 93 09 158 et FR 96 08 096

Cet appareil présente un transducteur de thérapie par émission d'ultrasons focalisés, qui est disposé dans une sonde rectale avec une enveloppe souple. La sonde présente une entrée et une sortie de fluide. Le fluide circulant à travers l'enveloppe souple de la sonde assure le refroidissement du transducteur de thérapie, assure aussi le refroidissement de la paroi rectale, et assure enfin la transmission des ultrasons depuis le transducteur vers les tissus. Dans le mode de réalisation actuel de l'appareil, l'enveloppe souple est un ballonnet à usage unique. Elle est fixée sur la sonde avant son introduction dans le patient. Les entrée et sortie de fluide de la sonde, qui sont situées dans la partie de la sonde qui reste en dehors du patient, sont en communication de fluide avec l'intérieur du ballonnet et permettent de gonfler plus ou moins ce ballonnet.

L'invention s'applique à la manipulation du fluide pour cet appareil. Elle propose une solution qui permet de résoudre un ou plusieurs de problèmes nouveaux suivants :
- isoler le fluide de l'environnement - patient ou extérieur -,
- contrôler la quantité de fluide appliqué à l'appareil ;
- récupérer le fluide après usage ;
- éviter les bulles, et
- contrôler la température, le débit et la pression du fluide.

Plus généralement, l'invention s'applique à d'autres types de dispositifs ou d'appareils que celui donné à titre d'exemple - une sonde avec un transducteur de thérapie - et pour lesquels on souhaite contrôler la distribution d'un fluide à récupérer. Elle s'applique généralement au contrôle de l'application d'un fluide à un dispositif qui présente une entrée de fluide et une sortie de fluide.

La solution de l'invention est simple et efficace; elle permet de résoudre les problèmes mentionnés plus haut.

On connaît également le brevet DE-19509005 C qui décrit un système de manipulation de fluide, comprenant un réservoir de fluide, un piège à bulles , un échangeur de chaleur et un appareil formant avec le piège à bulles et l'échangeur de chaleur un circuit fermé de fluide, et une pompe circulant le fluide dans le circuit fermé.

Dans le système connu, le réservoir est relié à la tubulure du circuit fermé par une vanne qui ouvre le circuit afin que le contenu du réservoir puisse se deverser dans la tubulure. Le piège à bulles n'est pas relié au réservoir, quelle que soit la position de la vanne.

L'invention propose un système de manipulation de fluide, elle propose aussi un kit de manipulation de fluide pour un appareil présentant une entrée et une sortie de fluide.

Selon l'invention, le système de manipulation de fluide, comprend un réservoir de fluide, un piège à bulles relié au réservoir, un échangeur de chaleur et un appareil formant avec le piège à bulles un circuit fermé de fluide, et une pompe circulant le fluide dans le circuit fermé.

Dans un mode de réalisation, la pompe est située en amont de l'appareil dans le sens de circulation du fluide.

Il est aussi préférable que l'échangeur de chaleur se trouve en amont de l'appareil dans le sens de circulation du fluide.

On peut prévoir une vanne entre le piège à bulles et le réservoir, un capteur de débit entre le piège à bulles et le réservoir, et/ou un capteur de débit sur le circuit fermé.

Dans un mode de réalisation préféré, l'appareil est une sonde ultrasonore de thérapie. Le fluide est alors un liquide de couplage et de refroidissement.

L'invention propose aussi un kit pour la manipulation de fluide vers un appareil présentant une entrée et une sortie, comprenant un réservoir de fluide, un piège à bulles, un échangeur de chaleur et des tubulures pour relier le réservoir au piège à bulles, et pour former un circuit fermé entre le piège à bulles, l'échangeur de chaleur et l'appareil.

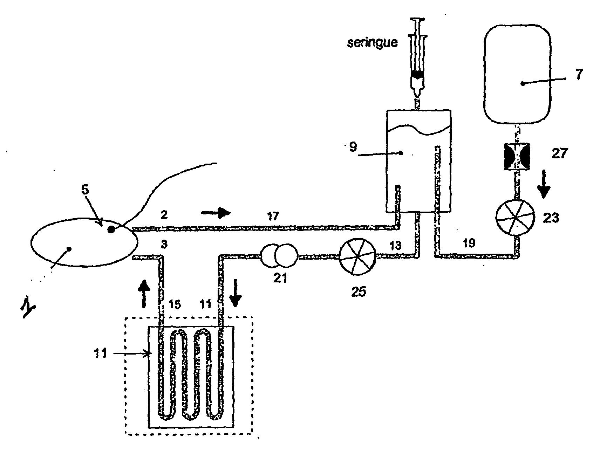

Dans un mode de réalisation préféré, le réservoir est scellé et est souple. D'autres caractéristiques et avantages de l'invention apparaîtront à la lecture de la description qui suit de modes de réalisation de l'invention, donnée à titre d'exemple et en référence aux dessins annexés dont la figure unique montre une représentation schématique d'un système de manipulation de fluide selon l'invention.

La figure montre schématiquement un appareil 1, qui présente une sortie de fluide 2 et une entrée de fluide 3, et à travers lequel la manipulation de fluide doit s'effectuer. Dans l'exemple, la sortie de fluide 2 se trouve en fonctionnement au-dessus de l'entrée de fluide, de sorte à améliorer l'évacuation des bulles avec le courant de fluide. L'appareil est par exemple une sonde rectale munie d'une enveloppe souple gonflable, comme celui mentionné plus haut. Dans ce cas, le fluide est par exemple le fluide de couplage ultrasonique décrit dans la demande de brevet français 9903738 de la demanderesse.

Dans la suite, l'invention n'est décrite qu'en référence à cet exemple, bien qu'il soit clair pour l'homme du métier qu'elle s'applique plus généralement à tout type d'appareil présentant une entrée de fluide et une sortie de fluide.

Outre l'appareil, le système de manipulation de fluide de l'invention comprend un réservoir de fluide 7. La sortie du réservoir de fluide est reliée à un piège à bulles 9. Ce piège à bulles fait partie d'un circuit fermé comprenant un échangeur de chaleur 11 et l'appareil 1.

Plus précisément, une sortie du piège à bulles 9 est reliée par une tubulure 13 à une entrée de l'échangeur de chaleur 11. Une sortie de l'échangeur de chaleur est reliée par une tubulure 15 à l'entrée de l'appareil, et la sortie de l'appareil est reliée par une tubulure 17 à une entrée du piège à bulles 9. L'homme du métier, dans cette description, comprendra que les termes "d'entrée" et de "sortie" désignent des orifices des divers éléments, et ne sont en fait dans la pratique différenciés que par le sens de circulation du fluide. Ainsi, dans l'exemple, le piège à bulles peut présenter trois orifices, reliés d'une part à la tubulure 13, d'autre part à la tubulure 17, et enfin à une tubulure 19 dont l'autre extrémité est reliée au réservoir. L'homme du métier comprendra aussi que les tubulures peuvent être monobloc avec certains des éléments, et par exemple, l'échangeur de chaleur pourrait être monobloc avec les tubulures 13 et 15.

La figure montre encore une pompe 21 permettant de circuler le fluide à travers le circuit fermé formé du piège à bulles, de l'échangeur de chaleur et de l'appareil. Cette pompe est dans le montage de l'exemple située entre le piège à bulles et l'échangeur de chaleur.

Sont en outre représentés à la figure des détecteurs de débit 23 et 25, disposés respectivement entre le réservoir et le piège à bulles, et entre le piège à bulles et la pompe. La figure montre encore une vanne 27, située entre le réservoir et le piège à bulles. On a enfin porté sur la figure un capteur de température 5, disposé dans l'appareil, dont la fonction apparaît dans la suite de la description.

Les modes de réalisation préférés des différents éléments de la figure sont maintenant décrits, avant de décrire le fonctionnement de l'invention.

Le réservoir 7 a pour fonction de contenir le fluide à manipuler. Il est de préférence constitué d'une pochette souple scellée. Ainsi il peut se vider sans avoir à pratiquer une entrée d'air; ceci permet d'éviter tout risque de regazage du fluide, et permet en outre un contrôle plus simple du volume. Le fluide est de préférence stérile pour des applications médicales, mais il pourrait s'agir aussi d'un fluide corrosif dans d'autres applications.

Une solution avantageuse pour relier le réservoir à une tubulure est de prévoir un orifice muni d'embout facile à percer lors de l'introduction d'une tubulure. Un tel embout est connu en tant que tel dans les applications médicales, et n'est pas décrit plus en détail. De préférence une chambre est située entre l'embout et la tubulure, permettant de piéger les bulles éventuelles.

En sortie du réservoir, sur la tubulure 19 entre le réservoir et le piège à bulles est prévu dans l'exemple une vanne. Celle-ci peut par exemple être constituée d'un organe de pincement de la tubulure, permettant d'arrêter la circulation de fluide, sans pour autant entrer en contact avec le fluide. La fonction de la vanne est simplement de permettre d'interrompre la liaison de fluide entre le réservoir et le piège à bulles.

Le piège à bulles 9 peut fonctionner suivant tout principe connu en soi. Dans la configuration la plus simple représentée sur la figure, le piège à bulles est simplement une enveloppe rigide située en un point haut du circuit, et dont les orifices sont dans la partie basse. Les bulles sont alors piégées dans la partie supérieure du piège à bulles. On peut aussi ajouter des raffinements, et par exemple assurer que la circulation de fluide dans le piège à bulles favorise le dégazage. Le piège à bulles se présente dans le mode de réalisation de la figure sous la forme d'un cylindre rigide en matière plastique transparente de hauteur environ 100 mm et de diamètre 25mm. Dans son fond sont situés 3 orifices prolongés à l'intérieur du cylindre par des portions de tubes. L'un de ces tubes monte jusqu'à 40 mm du fond, l'autre à 20 mm, tandis que le troisième affleure le fond. La tubulure 19 est connectée au premier orifiee, la tubulure 17 au second et la tubulure 13 au troisième. Ainsi l'air présent dans le piège à bulles n'est pas aspiré dans le circuit, tandis que les bulles contenues dans le circuit sont piégées. Le fait que la tubulure 19 soit reliée à l'orifice présentant la portion de tube la plus haute évite que le piège à bulle ne se vide complètement dans le réservoir, même si en cours de fonctionnement de la pompe le réservoir se trouve placé en dessous du piège à bulle.

On peut prévoir une valve située dans sa partie supérieure du piège à bulles pour permettre de vidanger le piège à bulle. Cette valve permet d'ôter l'air du piège à bulles, mais également d'ajouter ou d'enlever du liquide dans le circuit. Ce système présente avantageusement la caractéristique de sécurité de ne pouvoir être activé que par la mise en place d'une seringue, comme représenté sur la figure. En conséquence, en aucun cas la valve ne peut rester en position ouverte à l'air libre, ce qui préserve l'isolation du fluide. Un tel dispositif de sécurité est connu en soi, et est disponible commercialement sous le nom anglais de "syringe activated check valve", soit en langue française valve de sécurité activée par seringue.

La pompe 21 assure la circulation du fluide dans le circuit principal. Elle est de préférence de type péristaltique, et les tubulures y sont insérées. Ceci permet de ne pas contaminer le fluide et inversement, d'éviter que le fluide n'interagisse avec les éléments fixes de l'appareil. Ceci améliore encore l'isolation entre le fluide et l'environnement.

L'échangeur de chaleur 11 est dans l'exemple de réalisation de la figure formé d'un serpentin dans lequel circule le fluide et qui est plongé dans un bain thermostaté. Il peut être fabriqué en matière souple, telle que du PVC. Il peut aussi plus simplement être constitué d'une tubulure ou d'une partie de tubulure qui est immergée dans le bain thermostaté. Dans l'exemple d'un appareil d'hyperthermie, le fluide est refroidi, et le bain thermostaté est par exemple maintenu à une température voisine de 5°C de sorte que la température dans l'enveloppe de la sonde reste voisine de 15°C le débit étant autour de 8 litres / heure. Dans tous les cas, la fonction de l'échangeur de chaleur est de permettre d'agir sur la température du fluide qui le traverse. Cette température est dans l'exemple mesurée au niveau de l'appareil I, à l'aide du capteur de température 5. Son élément sensible est situé dans la zone à contrôler, par exemple dans l'enveloppe de la sonde à proximité de la sortie du fluide ou bien même dans la tubulure de sortie. Cette position du capteur est avantageuse en ce qu'elle permet aussi de détecter un défaut de circulation, par un échauffement du fluide. Le capteur peut être connecté à un appareil électronique de mesure. On notera que le capteur n'est pas indispensable au fonctionnement de l'invention, et permet simplement un meilleur contrôle de ce fonctionnement.

Les détecteurs de débit permettent aussi un meilleur contrôle du fonctionnement du système de l'invention, et peuvent aussi être supprimés. On utilise avantageusement un débitmètre à palettes, pour l'un ou l'autre des détecteurs de débit. On contrôle sa rotation par une barrière optique qui est connectée à un dispositif électronique capable de détecter et de compter les impulsions, qui correspondent au mouvement des palettes. La détection peut être du type "tout ou rien" ou il est possible de mesurer la vitesse de rotation des palettes et ainsi calculer le débit du fluide.

Le fonctionnement du système est maintenant décrit.

### Premier Remplissage de l'appareil.

Au départ, aucun fluide n'est présent dans le circuit. Le système est assemblé : les tubulures sont montées de sorte à former le circuit fermé, les tubulures sont placées dans la pompe péristaltique et le réservoir est relié au piège à bulles. Le cas échéant, le ou les détecteurs de débit sont disposé en regard de la barrière optique. L'échangeur de chaleur est placé dans le bain thermostaté. Dans le cas de l'Ablatherm, le ballonnet flexible est placé autour de la sonde.

Pour remplir le circuit, le réservoir contenant le fluide est placé en position haute - c'est-à-dire au-dessus du piège à bulles. Il est percé dans sa partie basse avec le perforateur. Si présente, la vanne est ouverte. Le fluide s'écoule par gravitation dans les tubulures vers le piège à bulles, et depuis celui-ci vers l'appareil 1. Pour que le remplissage s'effectue correctement et rapidement, le réservoir est par exemple situé à une hauteur donnée H au-dessus de l'appareil, typiquement 1m environ.

On peut interrompre à tout moment le vidage de fluide depuis le réservoir vers le circuit, en actionnant la vanne 27. On notera aussi que le réservoir peut contenir exactement le fluide nécessaire. Ou encore, sa contenance peut être limitée de telle sorte que l'appareil, s'il est extensible, ne peut pas éclater.

Lorsque le réservoir 7 s'est vidé, soit totalement, soit jusqu'à une marque prédéterminée, la pompe de circulation 21 est mise en route. Ceci provoque la circulation du fluide à travers le circuit fermé. L'air contenu dans les tubulures, 13, 15, 17, dans l'appareil 1 et dans l'échangeur de chaleur 11 est ainsi mis en circulation avec le fluide. Il est aspiré hors de l'appareil - notamment du fait de la position de la sortie de l'appareil - et se trouve piégé lors de son passage dans le piège à bulles. Dans le cas de l'Ablatherm, on enlève notamment tout l'air qui peut avoir été contenu dans l'appareil, entre le ballonnet et la sonde. Après quelques secondes de circulation la totalité de l'air résiduel des tubulures et de l'appareil est collecté dans le piège à bulles. De préférence l'air présent dans le piège à bulles est entièrement extrait grâce à la seringue. Le fait d'extraire l'air du piège à bulle permet de régler avec précision le volume exact de fluide dans le circuit fermé, et donc en fonctionnement la quantité de fluide dans l'appareil, connaissant le volume du piège à bulles, de l'échangeur de chaleur, et des différents éléments du circuit.

On arrive ainsi à remplir à l'aide du réservoir l'appareil, sans contamination possible du fluide, et sans contact du fluide avec l'environnement. Comme évoqué plus haut et expliqué maintenant en détail, on peut facilement grâce à l'invention régler le volume de fluide dans le circuit.

### Dégonflage de l'appareil.

Après remplissage de l'appareil et purge de l'air résiduel, il peut être nécessaire de le vider partiellement. Par exemple dans le cas de l'Ablatherm, il faut le dégonfler avant d'introduire la sonde rectale dans le patient. Cette opération est réalisée en abaissant le réservoir, le cas échéant après avoir ouvert la vanne. La pompe est stoppée car le sens de circulation va à l'inverse du sens de vidange. Le réservoir se remplit à nouveau avec le fluide de l'appareil et du piège à bulles, qui se vident. Lorsque le réservoir contient la quantité voulue de fluide, on ferme la vanne. Un dégonflage partiel est donc possible. On notera que l'on n'introduit pas de bulles dans le circuit lors de cette opération.

### Gonflage de l'appareil.

L'appareil peut aussi être rempli avec un volume de fluide connu et maîtrisé (typiquement 150 cm³ dans l'exemple d'appareil donné plus haut) pour éviter son éclatement.

Le réservoir est alors replacé en position haute, le cas échéant après avoir ouvert la vanne, et le liquide de couplage remplit à nouveau l'appareil. La maîtrise du volume de remplissage est encore plus simple si le réservoir a un volume tel que lorsqu'il est vide, l'appareil est rempli de la quantité correcte de fluide (150 cm³ dans l'exemple). La totalité de ces transferts de fluide étant toujours réalisés avec le même volume de fluide, le volume final de l'appareil au moment du traitement est toujours sous contrôle. La pompe peut être actionnée, soit pour accélérer le remplissage, soit pour augmenter la pression dans l'appareil, dans la limite du volume de fluide contenu dans le réservoir.

La vanne placée sur la tubulure située entre le réservoir et le piège à bulle permet ainsi de réduire volontairement le volume de fluide contenu dans l'appareil. Dans le cas de l'Ablatherm, certains patients présentent des ampoules rectales étroites (par suite d'un traitement par radiothérapie par exemple), et la vanne permet ainsi un meilleur contrôle du volume de fluide dans le circuit et donc du volume de la sonde rectale. Le capteur de débit 23, s'il existe, peut permettre de contrôler la quantité de fluide injectée dans le circuit.

### Circulation du fluide.

En fonctionnement normal, le fluide circule dans le circuit fermé comprenant l'appareil 1, le piège à bulles 9, et l'échangeur de chaleur 11. Ainsi le volume de l'appareil est constant. L'absence de robinet ou vanne sur les tubulures a l'avantage d'éviter toute fausse manipulation à l'installation (mauvais branchement) ou à l'utilisation (robinet laissé ouvert). Dans ce dernier cas une fuite du fluide ou une prise d'air pourrait avoir lieu et l'appareil pourrait se vider ou se remplir de manière incontrôlée. Dans le cas de l'Ablatherm, une fuite de l'appareil pourrait provoquer une grave lésion rectale du fait d'une élévation importante de température. L'absence de robinet ou de vanne sur le circuit fermé constitue un élément de sécurité notable de l'invention. La température du fluide peut être contrôlée en permanence, si l'on dispose d'un capteur de température. De préférence le flux est également contrôlé, à l'aide du capteur de débit 25.

Les bulles résiduelles et celles formées dans l'appareil - dans l'exemple de l'Ablatherm, ces bulles sont provoquées par l'action des ultrasons sur le fluide de couplage - sont collectées dans le piège à bulles en cours de traitement de telle sorte que le fluide ne se recharge pas en air. Le volume des bulles formées dans l'appareil est faible devant le volume total de fluide, et on peut considérer que le volume de fluide dans le circuit est constant.

### Purge de l'appareil.

Pour vider l'appareil et les tubulures on procède comme pour le dégonflage. Le fluide est récupéré dans le réservoir.

Le fonctionnement de l'invention, tel qu'il est décrit plus haut, fait apparaître les avantages suivants. L'invention permet d'assurer la circulation et le contrôle de température du fluide. Dans le cas de l'utilisation avec des transducteurs de thérapie, il s'agit d'assurer la circulation et le refroidissement du fluide servant de fluide de couplage autour du transducteur et en regard de l'interface tissulaire. Dans le domaine médical il est préférable de disposer d'un système consommable et fermé : comme on l'a vu, l'invention permet le remplissage ou la vidange de l'appareil, sans aucun contact avec l'extérieur. On assure ainsi que le fluide reste aseptique, ce qui évite tout problème s'il entrait en contact avec le tissu.

L'invention permet un remplacement rapide et simple des constituants du système, et notamment des tubulures, du piège à bulles, ou encore un changement simple du réservoir.

L'invention permet de résoudre les problèmes mentionnés plus haut. Le fluide peut être isolé de l'environnement. Dans l'exemple, l'appareil entourant les transducteurs et en contact avec l'interface tissulaire peut être rempli avec un fluide spécial dont la conservation demande des précautions. Par exemple dans le domaine médical, ce fluide sera stérile. Par exemple encore, dans le domaine acoustique, ce fluide sera purgé d'air. Dans d'autres domaines, le fluide sera corrosif. Avant utilisation, le fluide peut être contenu dans le réservoir 7 étanche. De préférence ce réservoir contient une dose suffisante pour une utilisation. Après utilisation le fluide peut être récupéré, sans risque de débordement, et toujours sans risque de contact.

A tout instant, le volume de fluide peut être connu; on peut aussi le faire varier à volonté. Les bulles issues de l'appareil et des tubulures peuvent être évacuées du circuit fermé. Le fluide contenu dans l'appareil peut être refroidi ou réchauffé, à une température contrôlée. Dans l'exemple, la fonction de refroidissement de la paroi rectale est critique pour la sécurité et sa performance peut être contrôlée en toute circonstance.

On notera en outre les avantages particuliers suivants ; la disposition de la pompe sur le circuit (en amont de l'appareil, en aval de la connexion du piège à bulles 9 au réservoir) évite que l'appareil ne se vide, dans l'hypothèse du pincement d'une tubulure, et ceci quel que soit l'endroit où a lieu ce pincement.

En effet, l'appareil ne peut pas se remplir et éclater si le réservoir contient exactement le volume nécessaire, lorsque le réservoir est rempli, il n'y a donc plus de fluide dans le réservoir. Si la tubulure est pincée entre le réservoir et l'appareil, le réservoir se met en dépression sous l'action de la pompe. S'il y a dans le circuit une valve de sécurité, elle tient avantageusement cette dépression afin que l'air ne rentre pas dans le circuit, ce qui risquerait de faire éclater l'appareil.

La vanne permet d'éviter une vidange intempestive de l'appareil dans le réservoir, dans le cas ou, en cours de traitement, le réservoir serait replacé en position plus basse que l'appareil ou que le piège à bulles, par inadvertance. La position de cette vanne sur les tubulures (entre le réservoir et le piège à bulle) ne peut pas conduire à un défaut de fonctionnement quel que soit son état ouvert ou fermé. Même en l'absence de vanne, le prolongement du tube relié à la tubulure 40 dans le piège à bulles assure que le piège à bulle ne peut pas se vider complètement, et donc qu'il reste toujours du fluide en circulation dans l'appareil; dans le cas de l'Ablatherm, ceci assure une protection en toutes circonstances de la paroi rectale du patient.

L'invention concerne aussi un kit de manipulation de fluide, pour un tel système. Le kit se présente sous une forme de blister, fermé par une membrane type "Tyvek" (marque déposée), que l'utilisateur vient encastrer sur un support mécanique rigide monté sur l'Ablatherm. Le kit comprend un réservoir de fluide, un échangeur de chaleur, un piège à bulles, et des tubulures pour relier ces différents éléments. D'autres éléments (ballonnet jetable à placer autour de la sonde, ligature pour fixer le ballonnet, gel de couplage acoustique, réflecteur optique pour détecter les mouvements du patient, seringue) peuvent aussi faire partie du kit mais ne sont pas décrits dans ce document. Ce kit permet de remplacer après usage les éléments du circuit en contact avec le fluide. On peut ensuite utiliser un nouvel appareil stérile, et limiter ainsi au maximum le risque d'infections nosocomiales.

Bien entendu, la présente invention n'est pas limitée aux exemples et modes de réalisation décrits et représentés, mais elle est susceptible de nombreuses variantes accessibles à l'homme de l'art. On pourrait ainsi appliquer l'invention à d'autres appareils. La position de l'échangeur de chaleur pourrait varier dans le circuit, et il pourrait se trouver en sortie de l'appareil cependant la position en amont de l'appareil permet de limiter les pertes thermiques dans le circuit. La position préférée de la pompe est représentée sur la figure; la pompe pourrait aussi, même si c'est moins avantageux, se trouver entre l'échangeur de chaleur et l'appareil, ou encore entre l'appareil et le piège à bulles. De même, la position préférée du détecteur de débit est représentée sur la figure; ce détecteur pourrait aussi être placé en aval de la pompe. Cependant sa position en amont de la pompe permet de vérifier l'intégrité du circuit - absence de pincement ou de fuites dans le ballonnet et son circuit retour - et offre donc une sécurité accrue du fonctionnement.

On n'a pas décrit en détail plus haut les dispositifs de contrôle du système. On peut prévoir que les capteurs et la pompe sont commandés par un même dispositif de commande, qui peut être celui de l'appareil.

## Revendications

1. Un système de manipulation de fluide, comprenant un réservoir (7) de fluide, un piège à bulles (9), un échangeur de chaleur (11) et un appareil (1) formant avec le piège à bulles et l'échangeur de chaleur (11) un circuit fermé de fluide, et une pompe (21) circulant le fluide dans le circuit fermé **caractérisé en ce que** le piège à bulles comporte trois orifices, un premier et un deuxième orifices faisant partie du circuit fermé de fluide et un troisième orifice étant relié au réservoir (7)

2. Le système de la revendication 1, **caractérisé en ce que** la pompe est située en amont de l'appareil (1) dans le sens de circulation du fluide.

3. Le système de la revendication 1 ou 2, **caractérisé en ce que** l'échangeur de chaleur se trouve en amont de l'appareil (1) dans le sens de circulation du fluide.

4. Le système de l'une des revendications précédentes, **caractérisé en ce qu'**il présente une vanne (27) entre le piège à bulles (9) et le réservoir (7).

5. Le système de l'une des revendications précédentes, **caractérisé en ce qu'**il présente un capteur de débit (23) entre le piège à bulles (9) et le réservoir (7).

6. Le système de l'une des revendications précédentes, **caractérisé en ce qu'**il présente un capteur de débit (25) sur le circuit fermé.

7. Le système de l'une des revendications précédentes, **caractérisé en ce que** l'appareil est une sonde ultrasonore de thérapie.

8. Le système selon l'une des revendications précédentes **caractérisé en ce que** le niveau du réservoir (7) par rapport au piège à bulles (9) est réglable.

9. Le système selon l'une des revendications précédentes **caractérisé en ce que** le piège à bulles comporte une valve de sécurité activée par seringue.

10. Le système selon l'une des revendications précédentes **caractérisé en ce que** l'appareil (1) est muni d'un capteur de température (5).

11. Un kit pour la manipulation de fluide vers un appareil présentant une entrée et une sortie, comprenant un réservoir (7) de fluide, un piège à bulles (9) à trois orifices, un échangeur de chaleur (11) et une tubulure (19) pour relier le réservoir au piège à bulles, et des tubulures (13,15,17) pour former un circuit fermé entre le piège à bulles, l'échangeur de chaleur et l'appareil (1).

12. Le kit de la revendication 11, **caractérisé en ce que** le réservoir est scellé.

## Patentansprüche

1. System zur Behandlung von Flüssigkeiten, bestehend aus einem Flüssigkeitsbehälter (7), einem Blasenabscheider (Blasenauffangbehälter)(9), einem Wärmetauscher (11) und einem mit dem Blasenabscheider und dem Wärmetauscher (11) einen geschlossenen Flüssigkeitskreislauf bildenden Apparat (1) sowie einer die Flüssigkeit im geschlossenen Kreislauf umwälzenden Pumpe (21), **dadurch gekennzeichnet, dass** der Blasenabscheider drei Kanäle umfasst, einen ersten und einen zweiten Kanal, welche beide Teil des Flüssigkeitskreislaufes sind, und einen dritten, mit dem Behälter (7) verbundenen Kanal.

2. System nach Anspruch 1, **dadurch gekennzeichnet, dass** sich die Pumpe bezogen auf den Apparat (1) stromaufwärts in Fließrichtung der Flüssigkeit befindet.

3. System nach Anspruch 1 oder 2, **dadurch gekennzeichnet, dass** sich der Wärmetauscher bezogen auf den Apparat (1) stromaufwärts in Fließrichtung der Flüssigkeit befindet.

4. System nach einem der vorgenannten Ansprüche, **dadurch gekennzeichnet, dass** zwischen dem Blasenabscheider (9) und dem Behälter (7) ein Ventil (27) vorgesehen ist.

5. System nach einem der vorgenannten Ansprüche, **dadurch gekennzeichnet, dass** zwischen dem Blasenabscheider (9) und dem Behälter (7) ein Messgerät (23) zur Messung der Durchflussleistung vorgesehen ist.

6. System nach einem der vorgenannten Ansprüche, **dadurch gekennzeichnet, dass** im geschlossenen Kreislauf ein Messgerät (25) zur Messung der Durchflussleistung vorgesehen ist.

7. System nach einem der vorgenannten Ansprüche, **dadurch gekennzeichnet, dass** der Apparat eine Therapiemaßnahmen dienende Ultraschallsonde ist.

8. System nach einem der vorgenannten Ansprüche, **dadurch gekennzeichnet, dass** das Niveau des Behälters (7) bezogen auf den Blasenabscheider (9) regulierbar ist.

9. System nach einem der vorgenannten Ansprüche, **dadurch gekennzeichnet, dass** der Blasenabscheider ein mittels einer Spritze aktiviertes Sicherheitsventil umfasst.

10. System nach einem der vorgenannten Ansprüche, **dadurch gekennzeichnet, dass** der Apparat (1) mit einem Temperaturmessgerät (5) versehen ist.

11. Ausrüstungssatz für die Behandlung von Flüssigkeiten in Richtung eines eine Eintritts- und eine Austrittsöffnung aufweisenden Apparates, bestehend aus einem Flüssigkeitsbehälter (7), einem Blasenabscheider (9) mit drei Wegen (Kanälen), einem Wärmetauscher (11) und einer Düse (19) zur Verbindung des Behälters mit dem Blasenabscheider, sowie Düsen (13, 15, 17) zur Bildung eines geschlossenen Kreislaufes zwischen dem Blasenabscheider, dem Wärmetauscher und dem Apparat (1).

12. Ausrüstungssatz nach Anspruch 11, **dadurch gekennzeichnet, dass** der Behälter dicht verschlossen ist.

## Claims

1. A fluid manipulating system, comprising a fluid reservoir (7), a bubble trap (9), a heat exchanger (11) and an apparatus (1) forming, with the bubble trap and the heat exchanger (11), a closed fluid circuit, and a pump (21) making the fluid flow in the closed circuit, **characterised in that** the bubble trap has three orifices, a first and a second orifice being part of the closed fluid circuit and a third orifice being connected to said reservoir (7)..

2. The system as claimed in claim 1, **characterized in that** the pump is located upstream of the apparatus (1) in the flow direction of the fluid.

3. The system as claimed in claim 1 or 2, **characterized in that** the heat exchanger is upstream of the apparatus (1) in the flow direction of the fluid.

4. The system as claimed in one of the preceding claims, **characterized in that** it has a valve (27) between the bubble trap (9) and the reservoir (7).

5. The system as claimed in one of the preceding claims, **characterized in that** it has a flowrate sensor (23) between the bubble trap (9) and the reservoir (7).

6. The system as claimed in one of the preceding claims, **characterized in that** it has a flowrate sensor (25) on the closed circuit.

7. The system as claimed in one of the preceding claims, **characterized in that** the apparatus is an ultrasound therapy probe.

8. The system according to one of the preceding claims, characterize in that the level of the reservoir (7) with respect to the bubble trap (9) is adjustable.

9. The system according to one of the preceding claims, **characterised in that** the bubble trap includes a syringe-operated safety valve.

10. The system according to one of the preceding claims, **characterised in that** the apparatus (1) is provided with a temperature sensor (5).

11. A kit for manipulating fluid toward an apparatus having an inlet and an outlet, comprising a fluid reservoir (7), a three-orifice bubble trap (9), a heat exchanger (11) and a tube (19) connecting the reservoir to the bubble trap, and a tube system (13, 15, 17) for forming a closed circuit between the bubble trap, the heat exchanger and the apparatus (1).

12. The kit as claimed in claim 11, **characterized in that** the reservoir is sealed.
